# EUROPÄISCHE PATENTSCHRIFT

(11) **EP 3 578 118 B1**
(45) Veröffentlichungstag und Bekanntmachung des Hinweises auf die Patenterteilung: **31.08.2022**
(21) Anmeldenummer: 18176003.4
(22) Anmeldetag: 05.06.2018
(51) Int. Cl.: A61B 17/29, A61B 18/14, A61B 17/00, A61B 18/00

(54) **CHIRURGISCHES INSTRUMENT**
SURGICAL INSTRUMENT
INSTRUMENT CHIRURGICAL

(43) Veröffentlichungstag der Anmeldung: 11.12.2019
(73) Patentinhaber: Erbe Elektromedizin GmbH, 72072 Tübingen (DE)
(72) Erfinder: Kirstgen, Udo, 72108 Rottenburg (DE); Amann, Marcus, 72336 Balingen (DE); Buntrock, Volker, 72762 Reutlingen (DE)
(74) Vertreter: Rüger Abel Patentanwälte PartGmbB

(56) Entgegenhaltungen:
- EP-A1- 2 845 548
- EP-A1- 2 845 550
- EP-A2- 2 659 848
- US-A1- 2014 012 290

## Beschreibung

Die Erfindung betrifft ein chirurgisches Instrument gemäß Anspruch 1.

Im Folgenden beschreibt der Begriff "distal" stets den vom Anwender entfernten Teil des Instruments oder Bauteils, der Begriff "proximal" beschreibt stets den zum Anwender hin gerichteten näher liegenden Teil des Instruments oder Bauteils.

Die EP 2 845 550 A1 sowie die EP 2 845 548 A1 beschreiben ein chirurgisches Instrument zum Koagulieren, Versiegeln und Durchtrennen von biologischem Gewebe, wie beispielsweise Blutgefäßen. Das Instrument weist zwei Branchen auf, die über Zug-/Schubelemente in einer Schwenkbewegung geschlossen werden können, wobei zwischen den Zug-/Schubelementen ein Messer längsverschieblich gelagert ist.

Aus der EP 2 574 299 A2 ist ein Instrument bekannt, das ein Werkzeug mit einer beweglichen und einer festen Branche aufweist, um Gewebe zu fassen und festzuklemmen sowie durch Bestromung zu koagulieren. Die Bewegung eines Betätigungshebels wird über ein Gestänge auf das Werkzeug übertragen. Das Gestänge erstreckt sich durch den Schaft. Das Gestänge wird durch eine im Querschnitt U-Profil förmige Antriebsstange und eine darin angeordnete Messerstange gebildet.

Die EP 2 361 316 A1 offenbart ein chirurgisches Instrument mit einer beweglichen Branche, die auf eine andere Branche hin beweglich ist um Gewebe zu fassen. Das Instrument weist einen Handgriff sowie einen Betätigungshebel auf. Die Bewegung des Betätigungshebels wird über ein Betätigungsgetriebe in eine Bewegung umgesetzt, die durch Zug/Schub und Schiebeelemente auf das Werkzeug übertragen werden, die sich durch den Schaft erstrecken.

Die US 8 632 539 B2 offenbart ein chirurgisches Instrument mit zwei Branchen und einem geschlitzten Messer in welchem Schlitz eine Zug/Schubstange angeordnet ist, über welche die Branchen betätigt werden können.

Die US 2013/0066303 A1 beschreibt ein Instrument mit zwei Branchen und einem Messer, wobei das Messer in Kanälen der Branchen längsverschieblich ist.

Die DE 10 2016 106 397 A1 beschreibt ein chirurgisches Instrument mit Branchen, die in einem Schaft gelagert sind, an welchem Kulissen zum Führen der proximalen Endabschnitte der Branchen ausgebildet sind, um diese von einer geöffneten in eine geschlossene Stellung zu bewegen. An den proximalen Endabschnitten der Branchen sind Betätigungszapfen angeordnet, die mit je einer Ausnehmung in einer Zug-/Druckstange in Eingriff stehen, um die Branchen zu schließen.

Die US 2010/0031977 A1 offenbart ein Instrument mit zwei Scherenhebeln, wobei an einem Scherenhebel zwei Klammern angeordnet sind, die dazu eingerichtet sind, die beiden Scherenhebel zusammen zu halten.

Aus der US 2014/0012290 A1 ist ein Instrument mit zwei schwenkbeweglich gelagerten Branchen bekannt, die über ein Zug-/Schubelement zu öffnen und zu schließen sind. Das Zug-/Schubelement weist an seinem distalen Ende dazu einen Zapfen auf, der durch in den proximalen Enden der beiden Branchen vorgesehene Kulisse ragt. Auf den beiden Enden des Zapfens sitzen Ringe, die in die Kulissen greifen und die Reibung auf dem Zapfen vermindern sollen. Dies führt allerdings zu sehr schlanken Zapfen oder umgekehrt sehr weiten Kulissen.

Ausgehend von diesem Stand der Technik ist es Aufgabe der vorliegenden Erfindung, ein verbessertes chirurgisches Instrument anzugeben.

Diese Aufgabe wird mit einem chirurgischen Instrument nach Anspruch 1 gelöst:

Das erfindungsgemäße chirurgische Instrument der Erfindung weist ein erstes Werkzeug (Maulwerkzeug) mit einer ersten Branche und einer zweiten Branche auf, von denen wenigstens eine Branche schwenkbeweglich gelagert ist. Die Branchen können auch als Klauen oder Maulteile bezeichnet werden. Das Instrument weist einen Schaft mit einem distalen Ende auf, an welchem das erste Werkzeug gehalten ist. Ein Zug-/Schubelement des erfindungsgemäßen chirurgischen Instruments ist über eine Koppelstruktur mit der wenigstens einen schwenkbeweglich gelagerten Branche gekoppelt, um die Branchen zu schließen. Das Zug-/Schubelement kann sich beispielsweise durch den Schaft erstrecken. Alternativ oder zusätzlich kann sich das Zug-/Schubelement beispielsweise neben dem Schaft erstrecken und/oder das Zug-/Schubelement kann den Schaft beispielsweise umgeben. Das erfindungsgemäße chirurgische Instrument zeichnet sich dadurch aus, dass die Kopplung zwischen Zug-/Schubelement und schwenkbeweglich gelagerter Branche einen Betätigungszapfen und eine Kopplungsbahn, insbesondere Kulissenbahn, aufweist, wobei der Betätigungszapfen zur Kopplung der schwenkbeweglich gelagerten Branche mit dem Zug-/Schubelement mit der Kopplungsbahn über eine Hülse in Eingriff steht, die auf dem Betätigungszapfen drehbar gelagert ist.

Zwischen dem Träger der Kopplungsbahn, und dem Träger des Betätigungszapfens wird über die Kopplungsbahn und den Betätigungszapfen eine Kraft von dem Zug-/Schubelement auf die schwenkbeweglich gelagerte Branche übertragen. Bevorzugt ist die Kopplungsbahn eine Kulissenbahn, die die Übersetzung der Translationsbewegung des Zug-/Schubelements in die Schwenkbewegung der schwenkbeweglich gelagerten Branche vorgibt.

Die auf dem Betätigungszapfen drehbar gelagerte Hülse rollt auf der Kopplungsbahn ab und gleitet auf dem Betätigungszapfen. In bevorzugten Ausführungsformen stehen der Betätigungszapfen und die eine Kulissenbahn bildende Kopplungsbahn über die an der Kulissenbahn abrollende Hülse in Eingriff, so dass eine Zug- oder Schubbewegung des Zug-/Schubelements in eine Schließ- oder Öffnungsbewegung der wenigstens einen Branche umgewandelt wird. Gegenüber einem Instrument, bei welchem ein Betätigungszapfen ohne Hülse auf einer Kopplungsbahn, insbesondere Kulissenbahn, gleitet, so dass der Betätigungszapfen an der Kopplungsbahn gleitreibt, kommt es bei dem erfindungsgemäßen Instrument zwischen der Hülse und der Kopplungsbahn, insbesondere Kulissenbahn, zu Rollreibung und zwischen dem Betätigungszapfen und der Hülse zu Gleitreibung. Bei vorgegebener Kopplungsbahn kann die Kontaktfläche zwischen Betätigungszapfen und Hülse, über welche Kontaktfläche Gleitreibung auftritt, auf Grund der runden Form der Hülse größer sein als zwischen einem Betätigungszapfen mit dem Außendurchmesser der Hülse, welcher auf der vorgegebenen Kopplungsbahn ohne Hülse unter Auftreten von Gleitreibung zwischen Betätigungszapfen und Kopplungsbahn gleitet.

Es hat sich gezeigt, dass das Maulwerkzeug des erfindungsgemäßen chirurgischen Instruments auf Grund der Hülse auch nach einer Vielzahl von Schließbewegungen der wenigstens eine Branche, beispielsweise 250 Schließbewegungen, immer noch leichtgängig ohne Beeinträchtigung durch reibungsbedingten Verschleiß betätigt werden kann. Insbesondere werden Umformungen oder Abrieb an dem Betätigungszapfen oder der Kopplungs- oder Kulissenbahn auf Grund der Hülse vermieden oder vermindert, die die Bedienbarkeit des ersten Werkzeugs beeinträchtigen könnten. Insbesondere wird Gratbildung oder Kerbenbildung an der Kopplungs- oder Kulissenbahn vermindert. Die Standzeit des Maulwerkzeugs ist damit erhöht. Mit der Hülse können Verluste durch reibungsbedingten Verschleiß und/oder Reibungsverluste vermindert werden, so dass auch eine hohe gewünschter Klemmkraft an der Spitze der Branchen noch mit akzeptabler Handkraft aufgebracht werden kann. Es werden Hülsen aus beschichtetem oder nicht beschichtetem Metall, insbesondere Edelstahl, oder Keramik bevorzugt.

Die auf dem Betätigungszapfen gelagerte Hülse weist erfindungsgemäß einen Boden, insbesondere einen geschlossenen Boden, auf. Die Hülse kann damit dünnwandiger gefertigt werden, da der Boden die Stabilität der Hülse erhöht. Auf diese Weise kommt es auch bei dünnwandigen Hülsen nicht zu einem Eindrücken der Kante der Hülse. Durch eine becherförmige Hülse, die einen geschlossenen Boden aufweisen kann, wird die Stabilität auch einer dünnwandigen Hülse, insbesondere der Kante deutlich erhöht.

Der Betätigungszapfen ist vorzugsweise konisch, insbesondere sich zu seinem freien Ende konisch verjüngend ausgebildet. Bevorzugt ist auch der von der Hülse begrenzte Raum konisch, insbesondere konusabschnittförmig. Wenn ein Boden der Hülse vorhanden ist, verjüngt sich der Innenraum der Hülse vorzugsweise konisch zum Boden. Die Außenform der Hülse ist vorzugsweise konusabschnittförmig oder zylindrisch.

Ein konus- oder konusabschnittförmiger Betätigungszapfen kann mit einer sich innen konisch verjüngenden Hülse optimal zusammenarbeiten, insbesondere wenn die Gleitreibungskraft gleichmäßig auf die Oberfläche des Zapfens bzw. die Innenfläche der Hülse übertragen wird. Es ergibt sich außerdem ein Vorteil beim Zusammenbau des chirurgischen Instruments. Bei dem den Betätigungszapfen tragenden Teil, beispielsweise der Branche kann es sich beispielsweise um ein Metallpulverspritzgussteil handeln. Dieses kann bei konisch ausgebildetem Betätigungszapfen besser aus der Spritzgussform gelöst werden. Wenn die Hülse ebenfalls konisch ausgebildet ist, ist es aufgrund der Becherform bzw. mit dem Boden möglich, sicherzustellen, dass die Hülse richtig herum aufgesteckt wird. Ansonsten könnte es sogar zu erhöhtem Abrieb oder erhöhter Verformung des Betätigungszapfens der Hülse und/oder der Kulissenbahn kommen.

Zwischen der Hülse und dem Betätigungszapfen ist vorzugsweise eine Beschichtung der Hülse und/oder des Betätigungszapfens wirksam. Die Beschichtung kann beispielsweise auf dem Betätigungszapfen, insbesondere nur auf dem Betätigungszapfen, angeordnet sein. Die Beschichtung bildet mit der unbeschichteten oder beschichteten Hülse und/oder dem beschichteten oder unbeschichteten Betätigungszapfen ein Reibpaar mit geringerer Gleitreibung als zwischen dem Grundmaterial der Hülse und dem Grundmaterial des Betätigungszapfens.

Zwischen dem Betätigungszapfen und der Kulissenbahn ist vorzugsweise kein festes, insbesondere pulverförmiges, flüssiges oder pastöses Schmiermittel wirksam. Auf ein solches Schmiermittel kann insbesondere dann ohne Beeinträchtigung der Betätigung des ersten Werkzeugs auch nach mehrmaliger Benutzung verzichtet werden, wenn auf dem Betätigungszapfen eine vorstehend beschriebene Hülse drehbar gelagert ist, um auf der Kulissenbahn abzurollen und die Kraft zwischen Kulissenbahn und Betätigungszapfen zu übertragen und/oder wenn der Betätigungszapfen und/oder die Kulissenbahn beschichtet sind.

Das chirurgische Instrument kann ein weiteres Werkzeug, insbesondere ein Messer oder eine Wasserstrahlsonde aufweisen.

Bevorzugt sind sowohl die erste Branche als auch die zweite Branche schwenkbeweglich gelagert.

In bevorzugten Ausführungsformen ist an dem distalen Ende des Zug-/Schubelements ein Koppelelement vorgesehen, welches eine erste Seitenwand und eine zweite Seitenwand aufweist, die einander gegenüberliegen. Die Seitenwände können Öffnungen aufweisen oder geschlossen sein. Die Seitenwände begrenzen einen Aufnahmeraum zwischen den Seitenwänden. Der Aufnahmeraum kann beispielsweise ein Kanal, ein Schlitz oder ein Spalt sein. Bevorzugt trägt die erste Seitenwand eine erste Kopplungsstruktur und die zweite Seitenwand trägt bevorzugt eine zweite Kopplungsstruktur. Die erste Kopplungsstruktur steht mit der ersten Branche und die zweite Kopplungsstruktur an der gegenüberliegenden Seitenwand steht mit der zweiten Branche in Eingriff. In dem Aufnahmeraum ist vorzugsweise ein weiteres Werkzeug, insbesondere ein Messer, zwischen den Kopplungsstrukturen in Längsrichtung des Schaftes angeordnet oder zwischen den Kopplungsstrukturen in Längsrichtung des Schaftes verschiebbar geführt. Das Koppelelement weist wenigstens eine Verbindung der beiden Seitenwände auf, um einen Kopplungskörper zu bilden. Das Koppelelement kann beispielsweise im Querschnitt U-förmig sein, wobei der Aufnahmeraum zwischen den beiden "Schenkeln" der U-Form ein Schlitz sein kann, um das Messer zu führen. Der Abschnitt des Aufnahmeraums, der von dem zweiten Werkzeug, insbesondere dem Messer, zwischen den äußeren Grenzen des zweiten Werkzeugs eingenommen wird, ist vorzugsweise ununterbrochen. Entsprechend muss das zweite Werkzeug, insbesondere das Messer kein Langloch in dem Bereich aufweisen, der in dem Aufnahmeraum geführt ist. Das zweite Werkzeug kann in dem Aufnahmeraum, der in dem Koppelelement zwischen den Seitenwänden bzw. zwischen den Kopplungsstrukturen ausgebildet ist, sicher in dem Koppelelement geführt werden.

In bevorzugten Ausführungsformen ist die Kulissenbahn an dem Koppelelement als Kopplungsstruktur ausgebildet und die schwenkbeweglich gelagerte Branche weist bevorzugt den der Kulissenführung zugeordneten Betätigungszapfen auf. Alternativ kann die Kulissenbahn an der schwenkbeweglich gelagerten Branche und der mit der Kulissenbahn in Eingriff stehende Betätigungszapfen an dem Koppelelement ausgebildet sein.

Die Kulissenführung, insbesondere eine an einer seitlichen Wand des Koppelelements angeordnete Kulissenführung kann beispielsweise durch zwei gegenüberliegende Wandflächen gebildet sein, die eine Ausnehmung, insbesondere eine Nut oder einen Schlitz begrenzen. Die Ausnehmung weist vorzugsweise einen Boden auf. Die Wandflächen bilden die Kulissenbahnen für die Umwandlung der Zugbewegung bzw. der Schubbewegung in eine entsprechende Schließbewegung oder Öffnungsbewegung der Branchen. Der an der Branche oder dem Koppelelement angeordnete Betätigungszapfen greift in die Ausnehmung ein und endet vorzugsweise in der Ausnehmung.

Die Kulissenbahn kann gekrümmt sein. Die Kulissenbahn ist vorzugsweise schräg zur Translationsbewegungsrichtung bzw. schräg zur Längsrichtung des Schaftes gestellt. Die Kulissenbahn hat vorzugsweise unterschiedlich schräg zur Translationsbewegungsrichtung bzw. schräg zur Längsrichtung des Schaftes gestellte Abschnitte. Bevorzugt weist die Kulissenbahn wenigstens einen Knick auf. Beispielsweise können gegenüberliegende Kulissenbahnen bzw. Wandflächen, einen Knick aufweisen, so dass diese eine Ausnehmung, insbesondere eine Nut festlegen, die einen Knick aufweist oder beschreibt. Auf diese Weise erfolgt die Bewegungsübertragung von dem Zug-/Schubelement auf die schwenkbare Branche während eines Bewegungsablaufs nacheinander mit zwei unterschiedlichen Übersetzungen, insbesondere Übersetzungsstufen, von der Offenstellung in die Geschlossenstellung der Branchen.

Die schwenkbeweglich gelagerte Branche ist vorzugsweise mittels eines Lagerzapfens an dem Schaft schwenkgelagert. Der Lagerzapfen kann beispielsweise an der Branche ausgebildet sein. Die wenigstens eine Branche ist vorzugsweise derart schwenkgelagert, dass die Branche beim Verschwenken keine Translationsbewegung in Bezug auf den Schaft ausführt.

In bevorzugten Ausführungsformen sind die erste Branche und die zweite Branche an dem Schaft schwenkgelagert, wobei die Schwenkachsen der Branchen zueinander versetzt sind.

Bevorzugt ist in oder an dem Schaft ein Kanal angeordnet, in welchem das proximale Ende der schwenkbeweglich gelagerten Branche geführt ist, so dass eine Bewegung des proximalen Endes der schwenkbaren Branche in Richtung quer zu einer Schwenkebene, in welcher das proximale Ende der schwenkbeweglich gelagerten Branche geschwenkt wird, begrenzt ist oder verhindert wird.

Die erste Branche und die zweite Branche des erfindungsgemäßen chirurgischen Instruments weisen vorzugsweise je einen elektrischen Anschluss zum Verbinden der ersten Branche und der zweiten Branche mit einem HF-Generator auf. Die erste Branche und die zweite Branche können vorzugsweise mit HF-Leistung beaufschlagt werden, um ein bipolares Instrument zur Thermofusion zu bilden.

Kombinationen eines oder mehrerer vorstehend beschriebener Merkmale mit vorstehenden beschriebenen Ausführungsformen bilden weitere bevorzugte Ausführungsformen. Weitere vorteilhafte Merkmale und Ausführungsformen des erfindungsgemäßen chirurgischen Instruments gemäß dem ersten Aspekt ergeben sich aus den Unteransprüchen sowie nachfolgender Beschreibung und den Figuren der Zeichnung.

Es wird, gemäß einem weiteren Aspekt, zudem ein chirurgisches Instrument gemäß Anspruch 1 angegeben, welches dadurch gekennzeichnet ist, dass die erste Branche und die zweite Branche schwenkbeweglich gelagert sind, wobei an dem distalen Ende des Zug-/Schubelements ein Koppelelement vorgesehen ist, das an gegenüberliegenden Seitenwänden des Koppelelements je eine Kopplungsstruktur aufweist, von denen eine Kopplungsstruktur mit einer Branche und die Kopplungsstruktur an der gegenüberliegenden Seitenwand mit der anderen Branche in Eingriff steht, wobei das Koppelelement zwischen den Seitenwänden einen Aufnahmeraum festlegt, in welchem ein weiteres Werkzeug zwischen den Kopplungsstrukturen in Längsrichtung des Schaftes angeordnet oder in Längsrichtung des Schaftes verschiebbar geführt ist. Bei dem chirurgischen Instrument ist zwischen Zug-/Schubelement und schwenkbeweglich gelagerten Branchen in Ausführungsbeispielen eine Kopplung mittels Betätigungszapfen hergestellt sein, auf denen je eine Hülse drehbar gelagert ist. Ausführungsbeispiele des chirurgischen Instruments können mit einem oder mehreren beliebigen Merkmalen des chirurgischen Instruments gemäß vorliegender Anmeldung weitergebildet sein.

Es zeigen:
Fig. 1 - eine Ausführungsform des erfindungsgemäßen chirurgischen Instruments in perspektivischer Prinzipdarstellung,
Fig. 2 - das erste Werkzeuge des Instruments nach Fig. 1 in perspektivischer Darstellung,
Fig. 3 - ein zu dem ersten Werkzeug nach Fig. 2 gehöriges Sockelteil des Schaftes in perspektivischer Darstellung,
Fig. 4 - eine Branche des ersten Werkzeugs des erfindungsgemäßen Instruments gemäß Fig. 2 in perspektivischer Darstellung mit einer Hülse zum Anordnen auf dem Betätigungszapfen der Branche,
Fig. 5a - eine Hülse des ersten Werkzeugs des erfindungsgemäßen Instruments gemäß Fig. 1 und 2 in perspektivischer Darstellung,
Fig. 5b - eine Ansicht eines Längsschnitts mittig durch die Hülse gemäß Fig. 5a,
Fig. 5c - eine ausschnittsweise Längsschnittansicht der Hülse gemäß Figuren 5a, 5b auf dem Betätigungszapfen der Branche gemäß Fig. 4,
Fig. 6a - ein Zug-/Schubelement des erfindungsgemäßen Instruments gemäß Figuren 1 bis 3 mit einem Koppelelement, in dem ein Messer als weiteres Werkzeug geführt ist,
Fig. 6b - das Koppelelement gemäß Figur 6a in einer perspektivischen Ansicht auf die andere Seite des Koppelelements, und
Fig. 7 - eine vergrößerte Ansicht perspektivischer ausschnittsweiser Ansicht des ersten Werkzeugs gemäß Fig. 2.

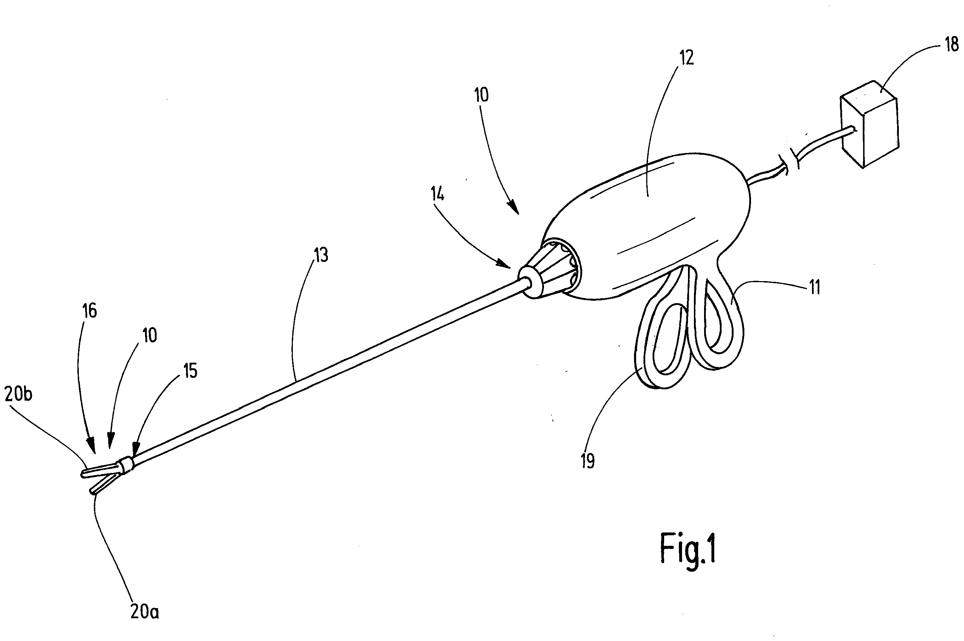

In Fig. 1 ist ein chirurgisches Instrument 10 veranschaulicht, das ein mit einem Handgriff 11 versehenes Gehäuse 12 aufweist. Das chirurgische Instrument 10 weist einen Schaft 13 auf, dessen proximales Ende 14 an dem Gehäuse 12 gehalten ist. Das distale Ende 15 des Schafts 13 trägt ein erstes Werkzeug 16 zur Einwirkung auf biologisches Material, beispielsweise zum Abklemmen, Koagulieren und Verschweißen. Durch den Schaft 13 erstreckt sich ein zweites Werkzeug 17, beispielsweise ein Messer 17 oder eine Fluidleitung, das dem nachfolgenden Trennen von Gewebe, z.B. Blutgefäßen, dient. Das chirurgische Instrument 10 ist bevorzugt an einen HF-Generator 18 angeschlossen. Bevorzugt handelt es sich bei dem erfindungsgemäßen chirurgischen Instrument 10 um ein bipolares Instrument zur Thermofusion.

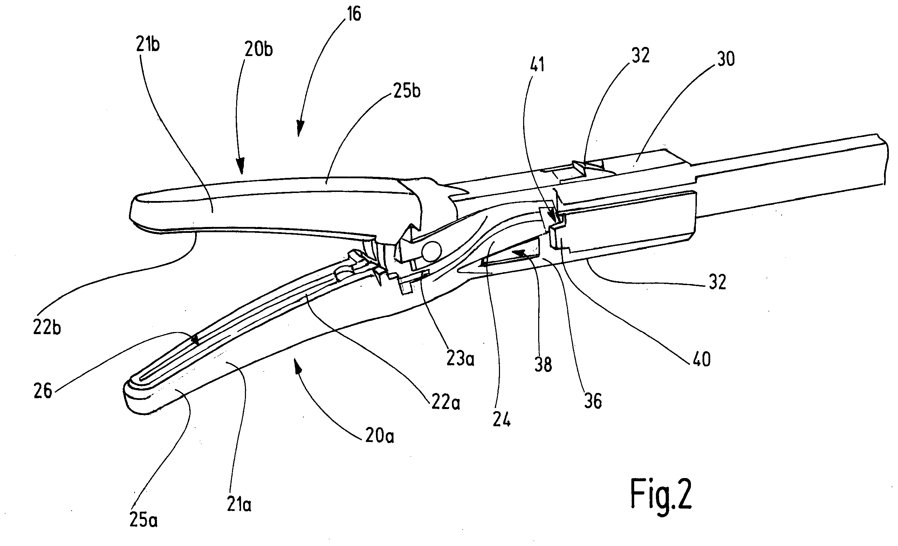

Das erste Werkzeug 16 ist in Fig. 2 gesondert veranschaulicht. Es weist eine erste Branche 20a und eine zweite Branche 20b auf, von denen mindestens eine, im vorliegenden Ausführungsbeispiel beide Branchen 20a, 20b um Schwenkachsen schwenkbeweglich gelagert sind. Jede Branche 20a, 20b besteht aus einem Branchenträger 21a, 21b und den darauf fixierten Elektrodeneinheiten 22a, 22b. Die Branchenträger 21a, 21b dienen dabei zur Übertragung der mechanischen Kräfte und zur Lagerung der Branchen 20a, 20b. Die Elektrodeneinheiten 22a, 22b sind über Anschlüsse 23a mit elektrische Leitungen verbunden und darüber mit HF-Generator 18 mit elektrischer HF-Leistung beaufschlagbar. Die elektrischen Leitungen sind in den Figuren der Übersichtlichkeit halber nicht dargestellt. Der Anschluss für die zweite Branche 20b ist in der Darstellung in Figur 2 verdeckt.

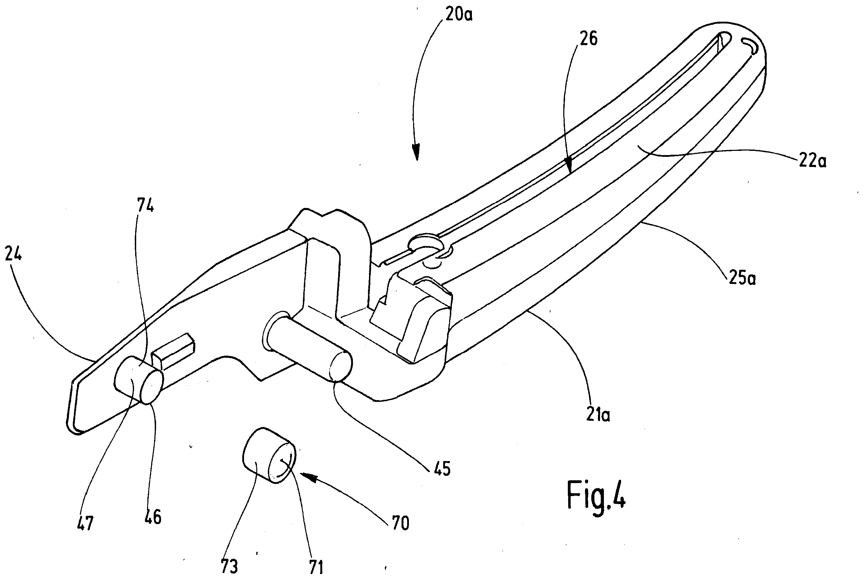

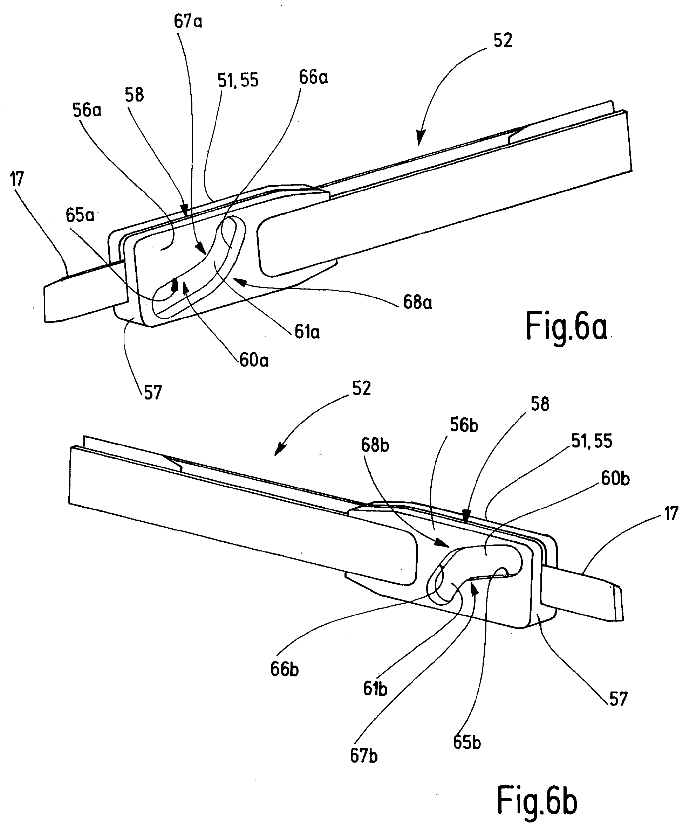

Wie insbesondere Fig. 4 erkennen lässt, hier am Beispiel der ersten Branche 20a erläutert, wobei selbiges auch für die zweite Branchen 20b gilt, weist der Branchenträger 21a einen Betätigungsabschnitt 24 und einen Trägerabschnitt 25a auf. Der Trägerabschnitt 25a trägt die Elektrodeneinheit 22a. In der Elektrodeneinheit 22a ist mittig ein beidseitig isolierter Kanal 26 oder eine Messerführungsnut vorgesehen, die zur Aufnahme und Führung eines Messers 17 (siehe insbesondere Fig. 6a, 6b) dient.

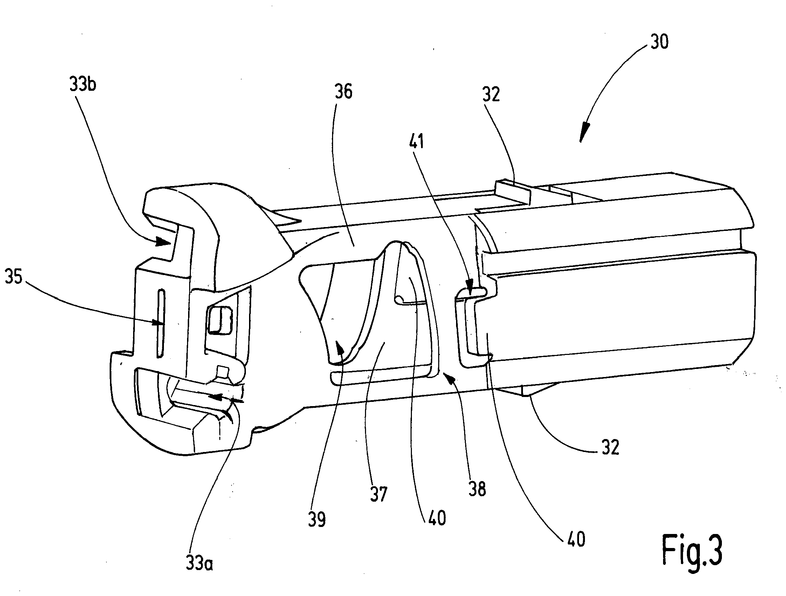

Der Schaft 13 weist einen distalen Abschnitt auf, der im vorliegenden Ausführungsbeispiel durch das Sockelteil 30 gebildet ist. Dieses ist in Figur 3 gesondert dargestellt. Das Sockelteil 30 kann beispielsweise aus Kunststoff hergestellt sein. Das Sockelteil 30 kann mit dem sich bis zum proximalen Ende des Schaftes 13 erstreckenden Rest des Schaftes über eine Rastverbindung verbunden sein. Das dargestellte Sockelteil 30 weist dazu Rastnasen 32 auf. Der Rest des Schaftes 13 ist in Fig. 2 nicht dargestellt. Die Branchen 20a, 20b des ersten Werkzeugs 16 sind an dem Sockelteil 30 schwenkbar gelagert. Dazu weist das Sockelteil 30 je eine Lagerausnehmung 33a, 33b auf. Die Lagerausnehmungen 33a, 33b sind voneinander beabstandet, so dass die Schwenkachsen nicht zusammenfallen, sondern bevorzugt parallel voneinander beabstandet sind. Somit kann das zweite Werkzeug 17 zwischen den Lagerausnehmungen 33a, 33b durch das Sockelteil 30 geführt werden. In dem vorliegenden Ausführungsbeispiel ist das Messer 17 durch das Sockelteil 30 geführt. Dafür ist in dem distalen Ende des Sockelteils ein Schlitz 35 angeordnet, um eine Führung für das Messer 17 zu bilden. Das Sockelteil 30 weist zwei gegenüberliegende Seitenwände 36, 37 auf, die zwischen ihnen einen Raum begrenzen. Die Seitenwände 36, 37 weisen je ein Fenster 38, 39 auf, wobei sich die Fenster 38, 39 gegenüberliegen. Das Sockelteil 30 weist zwei seitliche Fortsätze 40 auf. Die Fortsätze 40 bilden mit den Seitenwänden 36, 37 jeweils einen Kanal 41 bilden.

Die Branchen 20a, 20b weisen (siehe insbesondere Fig. 4 am Beispiel der ersten Branche 20a) zwischen dem Trägerabschnitt 25a und dem Betätigungsabschnitt 24 je einen Lagerzapfen 45 auf, der in der diesem zugeordneten Lagerausnehmung 33a, 33b in dem Sockelteil 30 angeordnet ist, um die Branche 20a, 20b schwenkbeweglich in dem Sockelteil 30 zu lagern. An den Betätigungsabschnitten 24 der Branchen 20a, 20b, in Figur 4 am Beispiel der ersten Branche 20a veranschaulicht, ist ein Betätigungszapfen 46 angeordnet. Der Betätigungszapfen 46 ragt durch das Fenster 38 in der Seitenwand 36 auf der entsprechenden Seite des Sockelteils 30. In dem durch die Seitenwände 36, 37 des Sockelteils 30 begrenzten Raum erstreckt sich längs durch den Raum ein Zug-/Schubelement 50 mit einem Koppelelement 51 (Koppelkörper), das mit einem Übertragungsabschnitt 52 des Zug-/Schubelements 50 verbunden, insbesondere verschweißt, oder nahtlos einstückig mit diesem ausgebildet ist. Während sich das Zug-/Schubelement 50 in der dargestellten Ausführungsform durch den Schaft 13 erstreckt, kann sich das Zug-/Schubelement 50 in anderen Ausführungsformen des erfindungsgemäßen Instruments 10 beispielsweise parallel neben dem Schaft 13 erstrecken oder kann beispielsweise rohrartig sein und den Schaft 13 umgeben.

Das Koppelelement 51 ist vorzugsweise ein Metallteil, insbesondere aus Edelstahl. Das Koppelelement 51 besteht aus einem Körper 55. Der Körper 55 der zwei Seitenwände 56a,b aufweist, die durch einen Boden 57 miteinander verbunden sind. In dem zwischen den Seitenwänden 56a, 56b festgelegten schlitzförmigen Aufnahmeraum 58 ist das zweite Werkzeug 17, im vorliegenden Ausführungsbeispiel ein Messer, längs verschieblich geführt. In Figuren 6a und 6b ist dies veranschaulicht. Die Figuren 6a und 6b zeigen zwei perspektivische Ansichten des Koppelelements 51, in dessen Aufnahmeraum 58 ein Messer geführt 17 ist. Das Messer 17 erstreckt sich durch den Aufnahmeraum 58 und in dem Übertragungsabschnitt 52.

Bevorzugt weist, wie in dem veranschaulichten Ausführungsbeispiel, jede Seitenwand 56a, b eine Nut 60a, 60b auf von denen auf Grund der Darstellung in Figur 6a nur die Nut 60a in einer Seitenwand 56a sichtbar ist. In diese Nut 60a greift der Betätigungszapfen 46 der ersten Branche 20a ein. Figur 6b zeigt das Koppelelement 51 von der anderen Seite mit der Nut 60b, in welche der Betätigungszapfen der zweiten Branche 20b eingreift. Die Nuten 60a, b weisen je einen Boden 61a, 61b auf. Der Aufnahmeraum 58 ist zwischen den Böden 61a, 61b angeordnet. Die Nuten 60a, 60b werden seitlich durch zwei gegenüberliegende Wandflächen begrenzt, die Kulissenbahnen 65a,b, 66a,b der Kulissenführung bilden, die in Gestalt der Nut 60a, 60b an jeder Seitenwand 56a, b ausgebildet ist. Anstatt die Kulissenbahnen 65a,b, 66a,b an dem Koppelelement 51 auszubilden, so dass das Koppelelement 51 der Träger der Kulissenbahnen 65a,b, 66a,b (Kurventräger) ist, und die Betätigungszapfen 46 an den schwenkbeweglichen Branche 20a, 20b ausgebildet sind, können umgekehrt die Kulissenbahnen 65a,b, 66a,b an den Branchen 20a, 20b und die Betätigungszapfen 46 an dem Koppelelement 51 angeordnet sein. Der Betätigungszapfen 46 der ersten Branchen 20a endet in der Nut 60a an der ersten Seitenwand 56a. Der nicht veranschaulichte Betätigungszapfen der zweiten Branche 20b endet entsprechend in der Nut 60b der zweiten Seitenwand 56b. Die Kulissenbahnen 65a, 66a an der ersten Seitenwand 56a arbeiten mit dem Betätigungszapfen 46 der ersten Branche 20a zusammen, um die Translationsbewegung des Zug-/Schubelements 50 in Längsrichtung des Schaftes 13 in eine Schwenkbewegung der Branche 20a umzusetzen. Entsprechendes gilt für den Betätigungszapfen der zweiten Branche 20b und die zugeordneten Kulissenbahnen 65b, 66b.

Die Kulissenbahnen 65a, 66a bzw. 65b, 66b weisen gegenüberliegende Knicke (s. Bezugszeichen 67a und 68a in Figur 6a sowie 67b und 68b in Figur 6b) auf, so dass eine Nut 60a, 60b mit einem Knick gebildet wird, an dem sich die Führungsrichtung verglichen mit einer Änderung der Führungsrichtung vor und nach dem Knick abrupt ändert. Die von den Kulissenbahnen 65a, 66a bzw. 65b, 66b vorgegebene Führungsrichtung hat an jedem Abschnitt der Kulissenbahnen 65a, 66a bzw. 65b, 66b eine Komponente mit der Zug- bzw. Schubrichtung entgegengesetzten Richtung. Der Knick führt zu einer unterschiedlichen Übersetzung der Translationsbewegung des Zug-/Schubelements 50 in eine Schwenkbewegung der Branche 20a, 20b bevor und nachdem der Betätigungszapfen 46 an dem Knick bzw. der Knick an dem Betätigungszapfen 46 vorbeigeführt wird. Die Abschnitte der Kulissenbahnen vor und nach dem Knick können gerade oder gekrümmt sein.

Wie in den Figuren 6a und 6b veranschaulicht ist das Messer 17 zwischen der Kopplungsstruktur mit den Kulissenbahnen 65a, 66a an der ersten Seitenwand 56a und der Kopplungsstruktur mit den Kulissenbahnen 65b, 66b an der zweiten Seitenwand 56b zwischen den Seitenwänden 56a, 56b geführt. Der Abschnitt des Aufnahmeraums 58, der zwischen den äußeren Kanten des Messers 17 von dem Messer eingenommen wird, ist nicht durch eine Verbindung, beispielsweise einen Steg, zwischen den Seitenwänden 56a, 56b unterbrochen. Auf Grund dessen muss das Messer 17 nicht notwendigerweise ein Langloch in dem Abschnitt des Messers 17 aufweisen, der durch das Koppelelement 51 geführt wird.

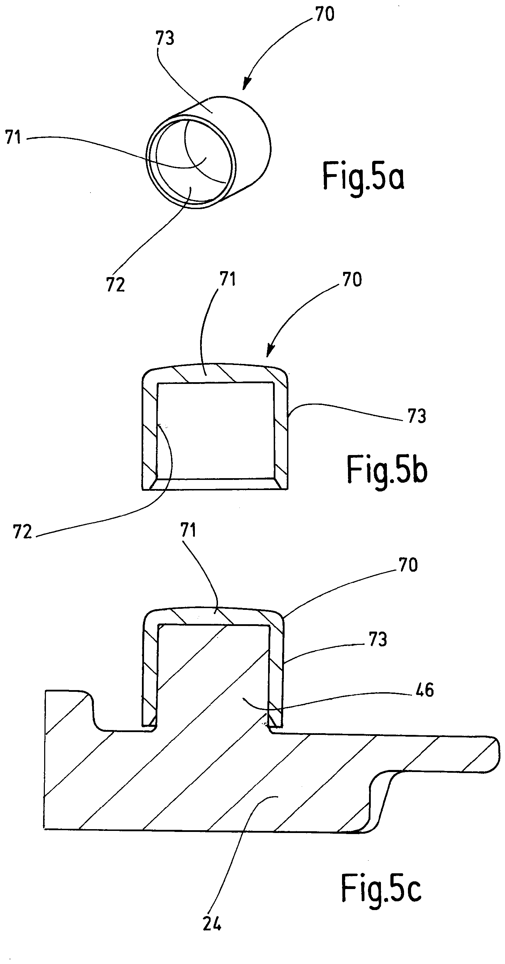

In Fig. 4 ist zusätzlich zu der perspektivischen Darstellung der ersten Branche 20a eine Hülse 70 perspektivisch dargestellt, die in dem zusammengesetzten ersten Werkzeug gemäß Fig. 2 auf dem Betätigungszapfen 46 drehbar gelagert angeordnet ist. Wie auch in den Fig. 5a, 5b, 5c veranschaulicht, weist die Hülse 70 vorzugsweise eine Becherform mit einem Boden 71 auf. Der Boden 71 ist, wie in der dargestellten Ausführungsform, vorzugsweise geschlossen. Andernfalls kann der Boden 71 beispielsweise aus Speichen bestehen. Der Boden 71 stabilisiert die Kante 72 der Hülse, an der der Boden 71 angeordnet ist. Die Hülse 70 kann mit dem Boden 71 dünnwandiger als ohne Boden 71 ausgeführt werden. Der Boden 71 außerdem stellt einen definierten Anschlag für den Betätigungszapfen 46 in Betätigungszapfenlängsrichtung dar (s. Fig. 5c, die eine Schnittdarstellung durch den Betätigungsabschnitt 24 durch den Betätigungszapfen 46 und die aufgesteckte Hülse 70 zeigt). Die Hülse 70 rollt bei einer relativen Bewegung des Betätigungszapfens 46 in der Nut 60a des Koppelelements 51 auf der der Translationsbewegung des Zug-/Schubelements entsprechenden Kulissenbahn 65a oder 65b ab, die durch die die Nut 60a begrenzenden Wandfläche gebildet ist. Die Hülse 70 gleitet dabei auf dem Betätigungszapfen 46. Um ein Abrollen der Hülse 70 auf der Kulissenbahn 65a oder 65b und ein Gleiten der Hülse 70 auf dem Betätigungszapfen 46 sicherzustellen, wird das Verhältnis des Außendurchmessers der Hülse 70 zum Innendurchmesser der Hülse 70 entsprechend gewählt. Die Böden 61a, 61b der Nuten 60a, 60b bilden eine Begrenzung für eine Bewegung der Hülsen 70 auf den Betätigungszapfen 46 in Längsrichtung der Betätigungszapfen 46.

Der Grundkörper der Hülse 70 ist vorzugsweise aus Metall, insbesondere Edelstahl, oder Keramik. Zwischen der Hülse 70 und dem Betätigungszapfen 46 ist vorzugsweise eine Beschichtung 74 zur Verminderung der Gleitreibung wirksam. Vorzugsweise ist der Betätigungszapfen 46 mit einer reibungsvermindernden Beschichtung 74 versehen. Alternativ oder zusätzlich kann auch die innere Umfangsfläche 72 der Hülse 70 mit einer gleitreibungsvermindernden Beschichtung beschichtet sein. Die reibungsvermindernde Beschichtung kann beispielsweise eine metallische, organische oder keramische Beschichtung sein. Die Beschichtung kann beispielsweise eine Diamond-like-Carbon-Schicht sein. Diamond-like Carbon bezeichnet diamantähnlichen amorphen Kohlenstoff.

Die äußere Mantelfläche 73 der Hülse 70 ist vorzugsweise zylindrisch. Der Innendurchmesser der Hülse kann sich in dem Abschnitt der inneren Umfangsfläche 72, der gleitend auf einem Abschnitt der äußeren Umfangfläche 47 des Betätigungszapfens 46 reibt, zum Boden 71 der Hülse 70 leicht konisch verjüngen. Ebenso kann sich der Betätigungszapfen 46 in dem Abschnitt der Umfangfläche 47, der mit der inneren Umfangsfläche 72 ein Reibpaar bildet, leicht konisch zu dem freien Ende des Betätigungszapfens 46 verjüngen. Die durch die konischen Formen des Betätigungszapfens 46 und des Inneren der Hülse 70 festgelegten Öffnungswinkel des Abschnitts der inneren Umfangsfläche 72 und des Abschnitts der Umfangfläche 47 können einander entsprechen. Ein konusabschnittförmiger Betätigungszapfen 46 kann mit einer sich innen konisch verjüngenden Hülse 70 optimal zusammenarbeiten, insbesondere wenn die Gleitreibungskraft gleichmäßig auf die Umfangfläche 47 des Betätigungszapfens 46 bzw. die Innere Umfangsfläche 72 der Hülse 70 übertragen wird. Es ergibt sich außerdem ein Vorteil beim Zusammenbau des chirurgischen Instruments 10. Bei den Branchen 20a, 20b kann es sich beispielsweise jeweils um ein Metallpulverspritzgussteil handeln. Dieses kann bei konisch ausgebildetem Betätigungszapfen 46 besser aus der Spritzgussform gelöst werden. Wenn die Hülse 70 innen ebenfalls konisch ausgebildet ist, ist es aufgrund der Becherform bzw. mit dem Boden 71 möglich, sicherzustellen, dass die Hülse 70 richtig herum auf den Betätigungszapfen 46 gesteckt wird, um erhöhten Abrieb oder erhöhte Verformung des Betätigungszapfens 46 der Hülse 70 und/oder der Kulissenbahn 65a,b bzw. 66a,b zu vermeiden.

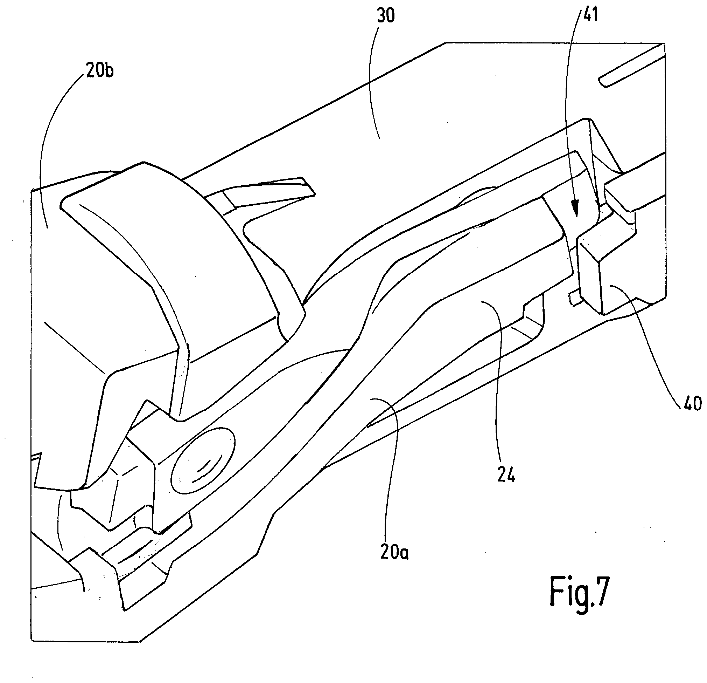

Das proximale Ende des Betätigungsabschnitts 24 der schwenkbeweglich gelagerten Branche ersten Branche 20a ist vorzugsweise zwischen dem Fortsatz 40, der sich an dem Sockelteil 30 des Schaftes 13 abstützt, und dem Sockelteil 30 in dem Kanal 41 geführt. Dies ist zusätzlich in Fig. 7 veranschaulicht, die eine vergrößerte Ausschnittsansicht des ersten Werkzeugs gemäß Fig. 2 zeigt. Dadurch wird eine Bewegung des proximalen Endes der Branche 20a quer zur Schwenkebene weg von dem Sockelteil 30 verhindert oder begrenzt. Dies fördert die Stabilität des erfindungsgemäßen Instruments 10.

Das insoweit beschriebene Instrument 10 arbeitet wie folgt:
In unbetätigtem Zustand sind die Branchen 20a, 20b voneinander weg gespreizt und das Messer 17 befindet sich in einer zurückgezogenen Position. Das Zug-/Schubelement 50 kann in dieser Stellung beispielsweise mittels einer Druckfeder (nicht dargestellt) in distaler Richtung vorgespannt sein, so dass die Branchen 20a, 20b gespreizt bleiben.

Betätigt der Anwender nun den Bedienhebel 19 am Handgriff 11 wird diese Betätigungsbewegung von einem in dem Gehäuse 12 angeordneten Getriebe in eine Zugbewegung umgesetzt, die auf das proximale Ende des Zug-/Schubelements 50 übertragen wird. Diese Zugbewegung in proximale Richtung schließt die Branchen 20a, 20b ggf. gegen die Kraft des Vorspannelements. Beim Schließen der Branchen 20a, 20b durch Zurückziehen des Zug-/Schubelements 50 in proximaler Richtung wird der Betätigungszapfen 46 an der Kulissenbahn 65a über die an dieser abrollende Hülse 70 geführt, wodurch die translatorische Bewegung des Zug-/Schubelements 50 in eine Schwenkbewegung der Branche 20a übertragen wird. Entsprechendes gilt für die zweite Branche 20b.

Ausgehend von vollständig geöffneten Branchen 20a, 20b werden diese beim Zurückziehen des Zug-/Schubelements 50 mit einer konstanten Geschwindigkeit zunächst mit einer ersten Winkelgeschwindigkeit geschlossen und nachdem der Betätigungszapfen 46 den Bereich in der Nut zwischen den Knickstellen durchlaufen hat, mit einer zweiten Winkelgeschwindigkeit geschlossen, wobei die zweite Winkelgeschwindigkeit kleiner ist als die erste Winkelgeschwindigkeit. Nach dem der Knick an dem Betätigungszapfen 46 vorbeigeführt worden ist, ist jedoch die Kraftübertragung größer als zuvor. Auf Grund des Knicks ist die Übersetzung der Translationsbewegung des Zug-/Schubelements 50 auf die beweglich gelagerten Branchen gestuft.

Bevorzugt bewirkt die translatorische Bewegung des Koppelelements 51 bzw. der Kulissenführung eine rotatorische Bewegung der Branchen 20a, 20b in einem Winkel von 0° bis ca. 25°. In Ausführungsbeispielen kann durch eine Betätigungskraft am Bedienhebel 19 von ca. 70 Newton am distalen Ende der Branchen 20a, 20b vorzugsweise eine Klemmkraft von ca. 40 Newton erreicht werden.

Bei einer derartig hohen Klemmkraft am Maulwerkzeug 16 wirkt, bedingt durch die einseitige Krafteinleitung in jede Branche 20a, 20b, auf die Branchen 20a, 20b ein Drehmoment in entgegengesetzte Richtung (asymmetrische Krafteinleitung). Daraus würden ohne die Hülsen 70 in der Kopplung zwischen Betätigungszapfen 46 und Koppelelement 51 hohe Reibkräfte resultieren. Die Hülsen 70 können jedoch an den Kulissenbahnen 65a, 65b abrollen und es wird somit weniger Reibung erzeugt. Damit ist der Wirkungsgrad zwischen Handkraft und Maulwerkzeugklemmung erhöht und es wird eine lange Standzeit des Werkzeugs 16 erreicht.

Die Branchen 20a, 20b fassen das zwischen ihnen liegende biologische Gewebe und halten es festgeklemmt. Das Gewebe kann mittels eines nicht weiter dargestellten Schalters und durch Aktivierung eines angeschlossenen Generators über die Elektrodeneinheiten 22a, 22b bestromt und koaguliert werden. Soll das koagulierte Gewebe nun beispielsweise getrennt werden, wird das Messer 17 von einem in dem Gehäuse angeordneten Mechanismus in distale Richtung bewegt. Es ist in dem Koppelelement 51 und dem Sockelteil 30 auch dann sicher geführt, wenn die Trägerabschnitte 25a, 25b, wie in Figur 2 und 4 veranschaulicht, weg von der Schwenkebene der Branchen gekrümmt sind. Auf Grund der Führung kann das Messer 17 seitlich nicht ausknicken, sondern wir sicher in der Messerführungsnut 26 und mit der nötigen Kraft durch denaturiertes Gewebe geführt, um dieses mit einem sauberen Schnitt sicher zu trennen.

Das erfindungsgemäße chirurgische Instrument 10 weist ein Maulwerkzeug 16 mit zwei Branchen 20a, 20b auf, die geschlossen werden können, um Gewebe zu greifen. Um die Kraft zum Schließen auf wenigstens eine Branche 20a, 20b zu übertragen, ist die Branche 20a, 20b mit einem vorzugsweise handbetätigbaren Zug-/Schubelement 50 gekoppelt, wobei zur Kopplung ein Betätigungszapfen 46, auf dem eine Hülse 70 gelagert ist, über die Hülse mit einer Kopplungsbahn 65a, b; 66a, b zusammen wirkt, wobei die Hülse auf der Kopplungsbahn 65a, b; 66a, b abrollt. Auf diese Weise wird Verformung, Abrieb oder Gratbildung an der Kopplungsbahn 65a, b; 66a, b in einem solchen Maß vermieden, dass auch nach einer Vielzahl von Schließbewegungen der Branche 20a, 20b diese noch immer unbeeinträchtigt von Abnutzung der Kopplungsbahn 65a, b; 66a, b bzw. des Betätigungszapfens 46 betätigt werden kann.

**Bezugszeichenliste:**

| | |
|---|---|
| 10 | Chirurgisches Instrument |
| 11 | Handgriff |
| 12 | Gehäuse |
| 13 | Schaft |
| 14 | Proximales Ende |
| 15 | Distales Ende |
| 16 | Erstes Werkzeug |
| 17 | Zweites Werkzeug |
| 18 | HF-Generator |
| 19 | Bedienhebel |
| 20a | Erste Branche |
| 20b | Zweite Branche |
| 21a | Branchenträger |
| 21b | Branchenträger |
| 22a | Elektrodeneinheiten |
| 22b | Elektrodeneinheiten |
| 23a | Anschluss |
| 24 | Betätigungsabschnitt |
| 25a | Trägerabschnitt |
| 25b | Trägerabschnitt |
| 26 | Kanal/Messerführungsnut |
| 30 | Sockel teil |
| 32 | Rastnasen |
| 33a | Lagerausnehmung |
| 33b | Lagerausnehmung |
| 35 | Schlitz |
| 36 | Seitenwand |
| 37 | Seitenwand |
| 38 | Fenster |
| 39 | Fenster |
| 40 | Vorsprung |
| 41 | Kanal |
| 45 | Lagerzapfen |
| 46 | Betätigungszapfen |
| 47 | Außenfläche |
| 50 | Zug-/Schubelement |
| 51 | Koppelelement |
| 52 | Übertragungsabschnitt |
| 55 | Körper |
| 56a,b | Seitenwände |
| 57 | Boden |
| 58 | Aufnahmeraum |
| 60a, b | Nut |
| 61a,b | Boden |
| 65a,b | Kulissenbahn |
| 66a,b | Kulissenbahn |
| 67a,b | Knick |
| 68a,b | Knick |
| 70 | Hülse |
| 71 | Boden |
| 72 | Innere Umfangsfläche |
| 73 | Äußere Mantelfläche |
| 74 | Beschichtung |

## Patentansprüche

1. Chirurgisches Instrument (10) mit einem ersten Werkzeug (16) mit einer ersten Branche (20a) und einer zweiten Branche (20b), von denen wenigstens eine Branche (20a, 20b) schwenkbeweglich gelagert ist, mit einem Schaft (13), der ein distales Ende (15) aufweist, an dem das erste Werkzeug (16) gehalten ist, mit einem Zug-/Schubelement (50), das über eine Koppelstruktur (46, 65a, b, 66a,b) mit der wenigstens einen schwenkbeweglich gelagerten Branche (20a, 20b) gekoppelt ist, um die Branchen (20a, 20b) zu schließen,
wobei
die Kopplung zwischen Zug-/Schubelement (50) und schwenkbeweglich gelagerter Branche (20a, 20b) einen Betätigungszapfen (46) und eine Kopplungsbahn (65a, b, 66a, b), insbesondere Kulissenbahn (65a, b; 66a, b) aufweist, wobei der Betätigungszapfen (46) zur Kopplung der schwenkbeweglich gelagerten Branche mit dem Zug-/Schubelement (50) mit der Kopplungsbahn (65a, b, 66a, b) über eine Hülse (70) in Eingriff steht, die auf dem Betätigungszapfen (46) drehbar gelagert ist
**dadurch gekennzeichnet, dass** die Hülse (70) einen Boden (71) aufweist.

2. Chirurgisches Instrument (10) nach einem der vorstehenden Ansprüche, wobei sich der Betätigungszapfen (46) konisch verjüngt und wobei sich die Hülse (70) innen konisch verjüngt.

3. Chirurgisches Instrument (10) nach einem der vorstehenden Ansprüche, wobei zwischen Hülse (70) und Betätigungszapfen (46) eine Beschichtung (74) der Hülse (70) und/oder des Betätigungszapfens (46) wirksam ist.

4. Chirurgisches Instrument (10) nach einem der vorstehenden Ansprüche, wobei zwischen der Kulissenbahn (65a, b; 66a, b) und dem Betätigungszapfen (46) kein festes, flüssiges oder pastöses Schmiermittel wirksam ist.

5. Chirurgisches Instrument (10) nach einem der vorstehenden Ansprüche, wobei die erste Branche (20a) und die zweite Branche (20b) schwenkbeweglich gelagert sind, wobei an dem distalen Ende des Zug-/Schubelements (50) ein Koppelelement (51) vorgesehen ist, das an gegenüberliegenden Seitenwänden (56a, b) des Koppelelements (51) je eine Kopplungsstruktur (65a,b, 66a,b) von denen eine Kopplungsstruktur (65a, 66a) mit einer Branche (20a) und die Kopplungsstruktur (65b, 66b) an der gegenüberliegenden Seitenwand (56 b) mit der anderen Branche (20b) in Eingriff steht, wobei das Koppelelement (51) zwischen den Seitenwänden einen Aufnahmeraum (58) festlegt, in welchem ein weiteres Werkzeug (17) zwischen den Kopplungsstrukturen (65a,b, 66a,b) angeordnet oder in Längsrichtung des Schaftes verschiebbar geführt ist.

6. Chirurgisches Instrument (10) nach einem der vorstehenden Ansprüche, wobei die Kulissenbahn (65a, b; 66a, b) wenigstens einen Knick (67a,b; 68a,b) aufweist, so dass die Bewegungsübertragung von dem Zug-/Schubelement (50) auf die schwenkbare Branche (20a, b) während eines Bewegungsablaufs nacheinander mit zwei unterschiedlichen Übersetzungen von der vollständig geöffneten Stellung in die geschlossene Stellung erfolgt.

7. Chirurgisches Instrument (10) nach einem der vorstehenden Ansprüche, wobei eine Kopplungsstruktur zur Kopplung des Zug-/Schubelements mit der schwenkbeweglich gelagerten Branche (20a, b) als Nut mit einem Boden (61a, b) ausgebildet ist, wobei die gegenüberliegenden Wandflächen, die die Nut begrenzen Kulissenbahnen (65a, b; 66a, b) bilden.

8. Chirurgisches Instrument (10) nach einem der vorstehenden Ansprüche, wobei die Kulissenbahn (65a, b; 66a, b) an dem Koppelelement (51) und der Betätigungszapfen (46) an der schwenkbeweglichen Branche (20a, b) ausgebildet ist.

9. Chirurgisches Instrument (10) nach einem der vorstehenden Ansprüche, wobei die schwenkbeweglich gelagerte Branche (20a, b) mittels eines Lagerzapfens (45) an dem Schaft (13) schwenkgelagert ist.

10. Chirurgisches Instrument (10) nach einem der vorstehenden Ansprüche, wobei die erste Branche (20a) und die zweite Branche (20b) an dem Schaft (13) schwenkgelagert sind und wobei die Schwenkachsen () der Branchen (20a, b) zueinander versetzt sind.

11. Chirurgisches Instrument (10) nach einem der vorstehenden Ansprüche, wobei das proximale Ende der schwenkbeweglich gelagerten Branche (20a, b) in einem Kanal (41) geführt ist, der in oder an dem Schaft (13) angeordnet ist, so dass eine Bewegung des proximalen Endes der schwenkbeweglich gelagerten Branche (20a,b) quer zur einer Schwenkebene der schwenkbeweglich gelagerten Branche (20a,b) begrenzt oder verhindert wird.

12. Chirurgisches Instrument (10) nach einem der vorstehenden Ansprüche, wobei die erste Branche (20a) und die zweite Branche (20b) je einen elektrischen Anschluss (23a) zum Verbinden der ersten Branche (20a) und der zweiten Branche (20b) mit einem HF-Generator (18) aufweisen.

## Claims

1. Surgical instrument (10) with a first tool (16) having a first branch (20a) and a second branch (20b), of which at least one branch (20a, 20b) is pivotably mounted, with a shank (13) having a distal end (15) on which the first tool (16) is held, with a push-pull element (50) which is coupled via a coupling structure (46; 65a, b; 66a, b) to the at least one pivotably mounted branch (20a, 20b) in order to close the branches (20a, 20b),
wherein
the coupling between the push-pull element (50) and the pivotably mounted branch (20a, 20b) comprises an actuating peg (46) and a coupling track (65a, b; 66a, b), in particular a sliding block track (65a, b; 66a, b), wherein for coupling the pivotably mounted branch to the push-pull element (50), the actuating peg (46) is in engagement with the coupling track (65a, b; 66a, b) via a sleeve (70) which is rotatably mounted on the actuating peg (46),
**characterised in that** the sleeve (70) has a base (71).

2. Surgical instrument (10) according to any of the preceding claims, wherein the actuating peg (46) tapers conically and wherein the sleeve (70) tapers conically on the inside.

3. Surgical instrument (10) according to any of the preceding claims, wherein a coating (74) of the sleeve (70) and/or of the actuating peg (46) is active between the sleeve (70) and the actuating peg (46).

4. Surgical instrument (10) according to any of the preceding claims, wherein there is no solid, liquid or paste-like lubricant active between the sliding block track (65a, b; 66a, b) and the actuating peg (46).

5. Surgical instrument (10) according to any of the preceding claims, wherein the first branch (20a) and the second branch (20b) are pivotably mounted, wherein a coupling element (51) is provided at the distal end of the push-pull element (50) and has a coupling structure (65a, b; 66a, b) on each opposite side wall (56a, b) of the coupling element (51), of which one coupling structure (65a, 66a) is in engagement with one branch (20a) and the coupling structure (65b, 66b) on the opposite side wall (56b) is in engagement with the other branch (20b), wherein the coupling element (51) establishes a receiving chamber (58) between the side walls, in which chamber a further tool (17) is arranged between the coupling structures (65a, b; 66a, b) or is guided so as to be displaceable in the longitudinal direction of the shank.

6. Surgical instrument (10) according to any of the preceding claims, wherein the sliding block track (65a, b; 66a, b) has at least one kink (67a, b; 68a, b), so that during a movement sequence, the movement transmission from the push-pull element (50) to the pivotable branch (20a, b) takes place successively with two different translation movements from the fully open position to the closed position.

7. Surgical instrument (10) according to any of the preceding claims, wherein a coupling structure for coupling the push-pull element to the pivotably mounted branch (20a, b) is configured as a groove with a base (61a, b), wherein the opposing wall faces which limit the groove form the sliding block track (65a, b; 66a, b).

8. Surgical instrument (10) according to any of the preceding claims, wherein the sliding block track (65a, b; 66a, b) is formed on the coupling element (51) and the actuating peg (46) is formed on the pivotable branch (20a, b).

9. Surgical instrument (10) according to any of the preceding claims, wherein the pivotably mounted branch (20a, b) is pivotably mounted on the shank (13) by means of a bearing journal (45).

10. Surgical instrument (10) according to any of the preceding claims, wherein the first branch (20a) and the second branch (20b) are pivotably mounted on shank (13), and wherein the pivot axes of the branches (20a, b) are offset to one another.

11. Surgical instrument (10) according to any of the preceding claims, wherein the proximal end of the pivotably mounted branch (20a, b) is guided in a channel (41) arranged in or on the shank (13), so that a movement of the proximal end of the pivotably mounted branch (20a, b) transversely to a pivot plane of the pivotably mounted branch (20a, b) is limited or prevented.

12. Surgical instrument (10) according to any of the preceding claims, wherein the first branch (20a) and the second branch (20b) each have an electrical connection (23a) for connection of the first branch (20a) and the second branch (20b) to an HF generator (18).

## Revendications

1. Instrument chirurgical (10) comprenant un premier outil (16) avec un premier mors (20a) et un deuxième mors (20b), parmi lesquels au moins un mors (20a, 20b) est monté avec possibilité de pivotement, comprenant une tige (13) qui présente une extrémité distale (15) sur laquelle est tenu le premier outil (16), comprenant un élément de traction/poussée (50) qui est couplé par l'intermédiaire d'une structure de couplage (46, 65a, b, 66a, b) au mors (20a, 20b), au nombre d'au moins un, monté avec possibilité de pivotement, en vue de fermer les mors (20a, 20b),
dans lequel
le couplage entre l'élément de traction/poussée (50) et le mors (20a, 20b) monté pivotant présente un doigt d'actionnement (46) et un chemin de couplage (65a, b, 66a, b), en particulier un chemin à coulisse (65a, b ; 66a, b), sachant que le doigt d'actionnement (46), en vue du couplage du mors monté pivotant avec l'élément de traction/poussée (50), est en prise avec le chemin de couplage (65a, b, 66a, b) par l'intermédiaire d'un manchon (70) qui est monté tournant sur le doigt d'actionnement (46),
**caractérisé en ce que** le manchon (70) présente un fond (71).

2. Instrument chirurgical (10) selon l'une des revendications précédentes, dans lequel le doigt d'actionnement (46) diminue en section de façon conique et le manchon (70) se rétrécit en section interne de façon conique.

3. Instrument chirurgical (10) selon l'une des revendications précédentes, dans lequel un revêtement (74) du manchon (70) et/ou du doigt d'actionnement (46) est actif entre le manchon (70) et le doigt d'actionnement (46).

4. Instrument chirurgical (10) selon l'une des revendications précédentes, dans lequel aucun lubrifiant solide, liquide ou pâteux n'est actif entre le chemin à coulisse (65a, b ; 66a, b) et le doigt d'actionnement (46).

5. Instrument chirurgical (10) selon l'une des revendications précédentes, dans lequel le premier mors (20a) et le deuxième mors (20b) sont montés pivotants, dans lequel il est prévu à l'extrémité distale de l'élément de traction/poussée (50), un élément de couplage (51) qui, sur des parois latérales (56a, b) opposées de l'élément de couplage (51), respectivement une structure de couplage (65a, b, 66a, b), parmi lesquelles une structure de couplage (65a, 66a) est en prise avec un mors (20a) et la structure de couplage (65b, 66b) sur la paroi latérale (56b) opposée est en prise avec l'autre mors (20b), l'élément de couplage (51) définissant entre les parois latérales un espace (58) dans lequel un outil (17) supplémentaire est disposé entre les structures de couplage (65a, b, 66a, b) ou est guidé avec possibilité de coulissement dans le sens longitudinal de la tige.

6. Instrument chirurgical (10) selon l'une des revendications précédentes, dans lequel le chemin à coulisse (65a, b ; 66a, b) présente au moins un coude (67a, b ; 68a, b), de manière à ce que pendant le déroulement d'un mouvement, la transmission du mouvement de l'élément de traction/poussée (50) au mors (20a, b) pivotant s'effectue successivement avec deux rapports de transmission différents, de la position entièrement ouverte à la position fermée.

7. Instrument chirurgical (10) selon l'une des revendications précédentes, dans lequel une structure de couplage est réalisée sous forme de rainure dotée d'un fond (61a, b), en vue du couplage de l'élément de traction/poussée avec le mors (20a, b) monté pivotant, sachant que les surfaces de paroi opposées, qui délimitent la rainure, forment des chemins à coulisse (65a, b ; 66a, b).

8. Instrument chirurgical (10) selon l'une des revendications précédentes, dans lequel le chemin à coulisse (65a, b ; 66a, b) est réalisé sur l'élément de couplage (51) et le doigt d'actionnement (46) sur le mors (20a, b) pivotant.

9. Instrument chirurgical (10) selon l'une des revendications précédentes, dans lequel le mors (20a, b) monté avec possibilité de pivotement est monté pivotant au moyen d'un tourillon (45) sur la tige (13).

10. Instrument chirurgical (10) selon l'une des revendications précédentes, dans lequel le premier mors (20a) et le deuxième mors (20b) sont montés pivotants sur la tige (13), et les axes de pivotement () des mors (20a, b) sont décalés l'un par rapport à l'autre.

11. Instrument chirurgical (10) selon l'une des revendications précédentes, dans lequel l'extrémité proximale du mors (20a, b) monté pivotant est guidée dans un canal (41) qui est disposé dans ou sur la tige (13), de manière à ce qu'un mouvement de l'extrémité proximale du mors (20a, b) monté pivotant, transversalement à un plan de pivotement du mors (20a, b) monté pivotant soit limité ou empêché.

12. Instrument chirurgical (10) selon l'une des revendications précédentes, dans lequel le premier mors (20a) et le deuxième mors (20b) présentent chacun une connexion électrique (23a) destinée à raccorder le premier mors (20a) et le deuxième mors (20b) à un générateur HF (18).
